# EUROPEAN PATENT SPECIFICATION

(11) **EP 2 429 412 B1**
(45) Date of publication and mention of the grant of the patent: **28.01.2015**
(21) Application number: 10714790.2
(22) Date of filing: 15.04.2010
(51) Int. Cl.: A61B 17/064

(54) **ROTARY TACK**
DREHREISSNAGEL
AGRAFE ROTATIVE

(30) Priority: 22.04.2009 US 427780
(43) Date of publication of application: 21.03.2012
(73) Proprietor: Easylap Ltd., 73150 Kfar Truman (IL)
(72) Inventor: RIMER, Ofer, 73150 Kfar Truman (IL); ALTMAN, Nir, 90912 Kfar Etzion (IL); LEVIN, Shalom, 30300 Atlit (IL); LEVIN, Asaf, 30300 Atlit (IL); FABIAN, Izhak, 73150 Kfar Truman (IL); COHEN, Arik, 30900 Zichron Ya'acov (IL)
(74) Representative: Lecomte & Partners
(86) International application number: PCT/US2010/031136
(87) International publication number: WO 2010/123740

(56) References cited:
- EP-A1- 0 663 184
- WO-A1-00/69345
- WO-A2-03/034925
- WO-A2-2004/112841
- WO-A2-2008/010948
- WO-A2-2008/038276
- GB-A- 2 417 208

## Description

### FIELD OF THE INVENTION

The present invention relates generally to rotary tacks, such as those used for hernia repairs and the like in laparoscopic and endoscopic procedures.

### BACKGROUND OF THE INVENTION

Tacks or other tissue fasteners made of resorbable or biodegradable materials (the terms being used interchangeably throughout) are well known. For example, US Patents 4884572 and 4895148 to Bays et al., describe repair tacks of biodegradable material chosen to have a degradation time in excess of the required healing time for the tissue.

EP 0 663 184 A1 and WO 00/69345 disclose a tack according to the preamble of claim 1. WO 2008/010948 A2 discloses a surgical fastener having a barrel-shaped body comprising a throughbore and including a helical thread.

### SUMMARY OF THE INVENTION

The present invention provides a rotary tack according to appended claims 1.

In accordance with one embodiment of the present invention the inner perimeter includes two straight portions that subtend an angle corresponding to two sides of a regular polygon and a single arcuate portion that extending from end points of the two straight portions. The regular polygon may be a pentagon.

In accordance with one embodiment of the present invention the arcuate portion and the straight portion are joined by a rounded corner. For example, the arcuate portion and the straight portions are joined by rounded corners and the two straight portions are joined by a rounded corner.

In accordance with one embodiment of the present invention an upper surface of the helix proximate the tip includes one or more steps.

### BRIEF DESCRIPTION OF THE DRAWINGS

The present invention will be understood and appreciated more fully from the following detailed description taken in conjunction with the drawings in which:
Figs. 1A-1D are simplified pictorial illustrations of a rotary tack, which is not in accordance with an embodiment of the present invention, wherein Figs. 1C and 1D are respectively bottom and top views; and
Figs. 2A-2C are simplified pictorial illustrations of a rotary tack, constructed and operative in accordance with an embodiment of the present invention, wherein Figs. 2B and 2C are respectively bottom and top views.

### DETAILED DESCRIPTION OF EMBODIMENTS

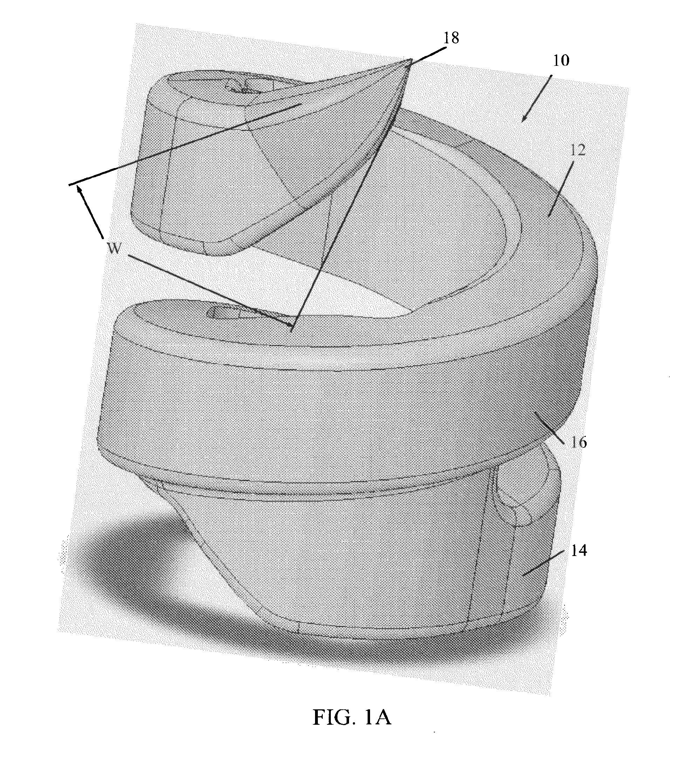

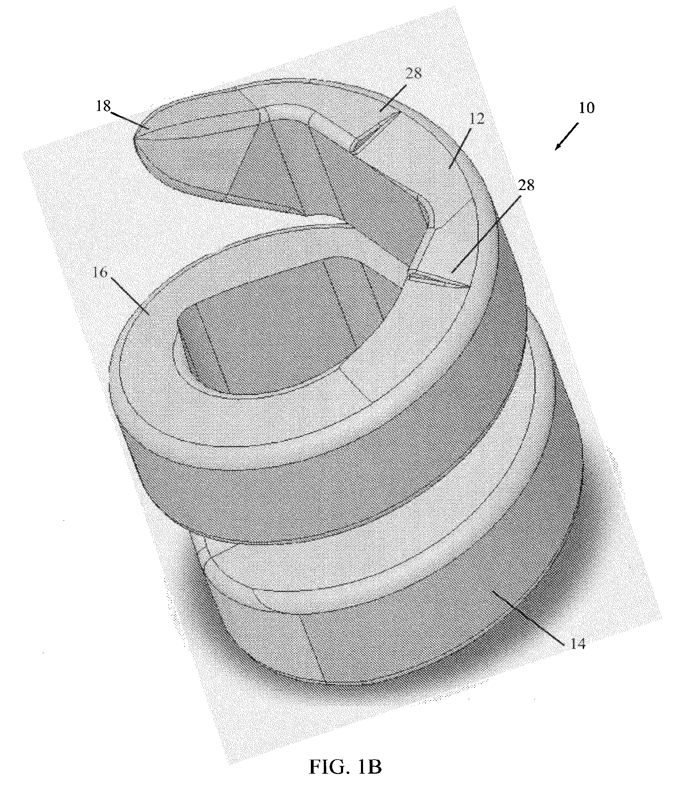

Reference is now made to Figs. 1A-1D, which illustrate a rotary tack 10, which is an example not in accordance with the present invention.

The tacks of the present invention are constructed of a resorbable material. The tacks may be constructed, without limitation, from a biodegradable polymer or copolymer of a type selected in accordance with the desired degradation time. That time, in turn, depends upon the anticipated healing time for the cartilaginous or other tissue which is the subject of the surgical procedure. Known biodegradable polymers and copolymers range in degradation time from about three months for polyglycolide to about forty-eight months for polyglutmic-co-leucine. A common biodegradable polymer used in absorbable sutures and the like is poly(L-lactide) which has a degradation time of about twelve to eighteen months. Without limitation, the tacks may be constructed from an absorbable copolymer derived from glycolic and lactic acids, such as a synthetic polyester chemically similar to other commercial available glycolide and lactide copolymers. Glycolide and lactide, in vivo, degrade and absorb by hydrolysis into lactic acid and glycolic acid which are then metabolized by the body.

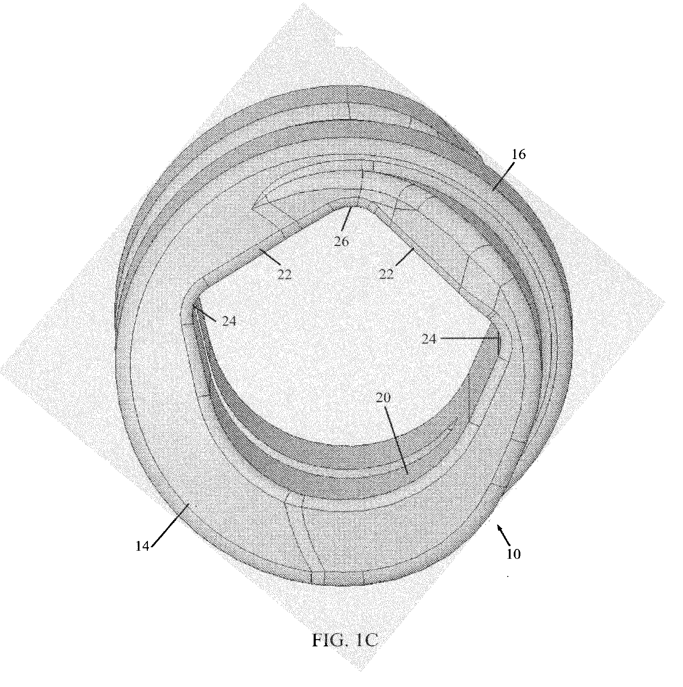

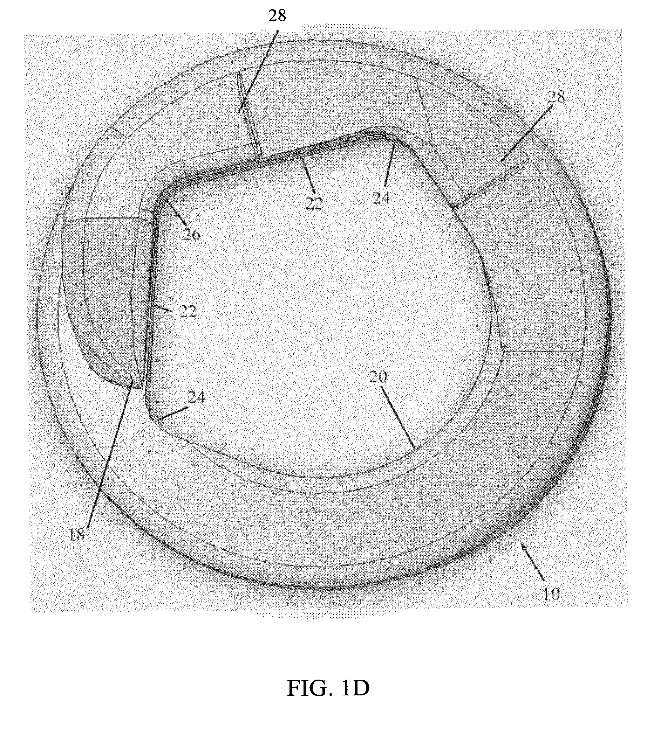

Tack 10 includes a helical body 12 having a closed-loop base 14 and a helix of spiral coils 16 that extend from base 14 and which terminate in a tip 18 for piercing tissue (not shown). As seen in Figs. 1C and 1D, each of the coils 16 has a cross-section with an inner perimeter including at least one arcuate portion 20 and at least one straight portion 22 contiguous to each other. In prior art helical fasteners, the base is open, that is, the lowermost coil is not a continuous closed coil or loop. In contrast, in the present invention, base 14 is closed. This geometry provides tack 10 with superior strength, especially important for use with the resorbable material.

According to Figs. 1A-1D, the inner perimeter includes two straight portions 22 that subtend an angle corresponding to two sides of a regular polygon and a single arcuate portion 20 that extending from end points of the two straight portions 22. The regular polygon may be a pentagon, wherein the subtended angle is 72°. The arcuate portion 20 and the straight portions 22 may be joined by a rounded corner. For example, the arcuate portion 20 and the straight portions 22 are joined by rounded corners 24 and the two straight portions are joined by a rounded corner 26. The inner perimeter comprises the drive section of tack 10, that is, the tack is placed on a complementary shaped drive shaft (not shown) which turns the tack in order to screw it into tissue (not shown).

As seen in Fig. 1A, the tip 18 has a sharp edge at a wide angle W aimed upwards relative to the helix. Angle W is much greater than the helix angle of the coils.

In accordance with one embodiment of the present invention an upper surface of the helix proximate tip 18 includes one or more steps 28 (this option not shown in Fig. 1A). Steps 28 may help prevent opening of the helix when pulling forces are applied to the tack 10.

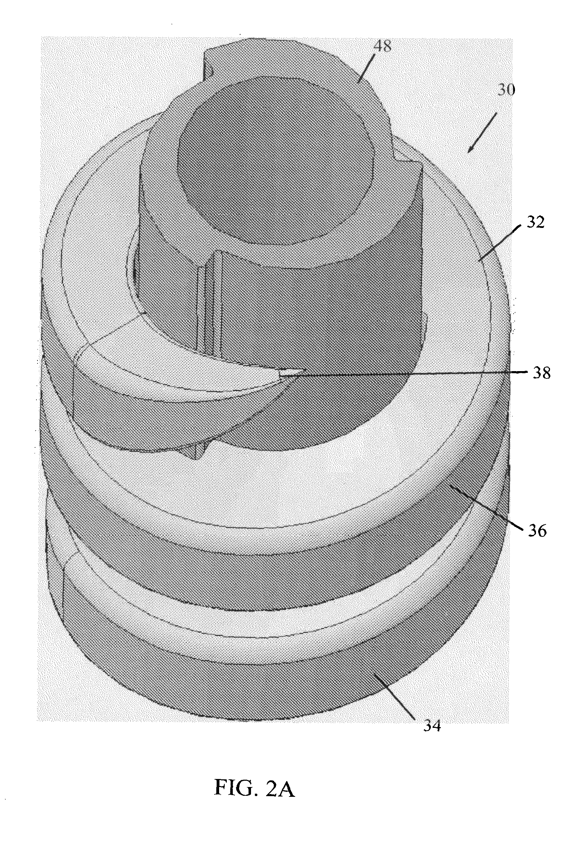

Reference is now made to Figs. 2A-2C, which illustrate a rotary tack 30, constructed and operative in accordance with an embodiment of the present invention.

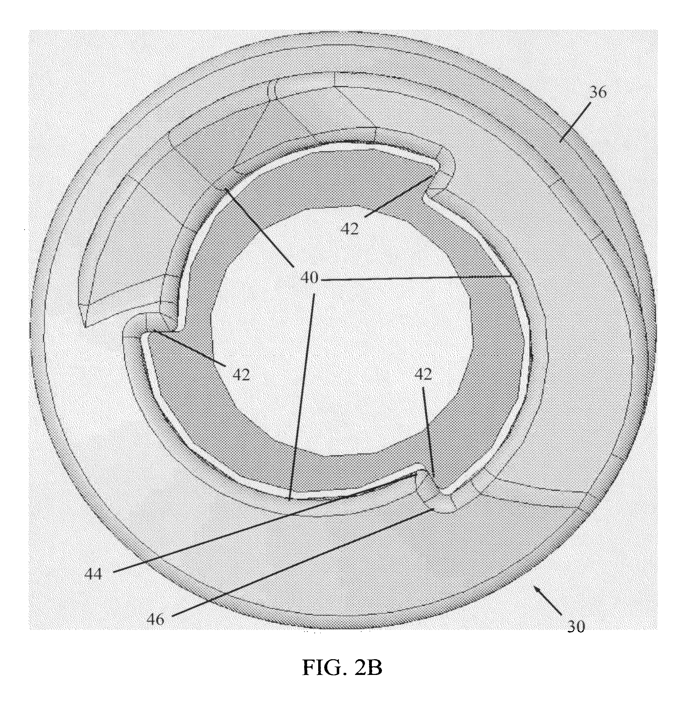

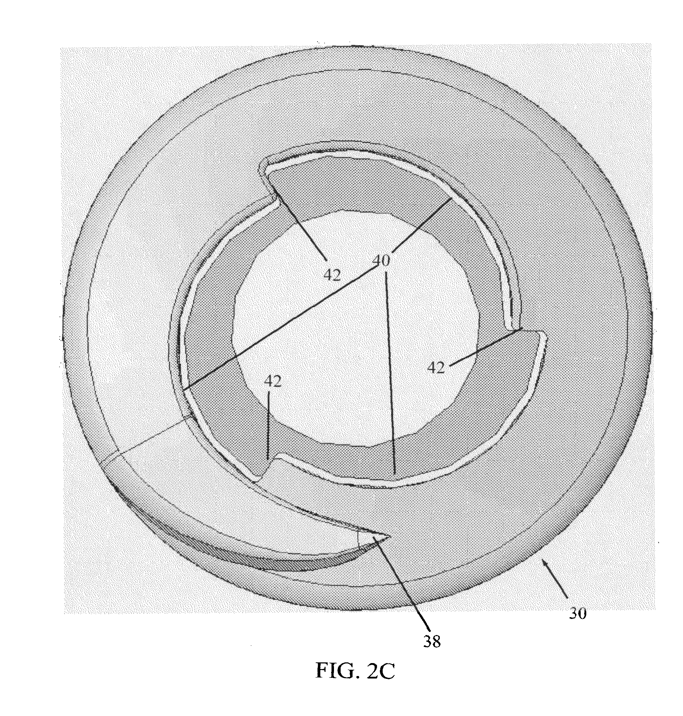

Tack 30 includes a helical body 32 having a closed-loop base 34 and a helix of spiral coils 36 that extend from base 34 and which terminate in a tip 38 for piercing tissue (not shown). As seen in Figs. 2B and 2C, each of the coils 36 has a cross-section with an inner perimeter including at least one arcuate portion and at least one straight portion contiguous to each other.

In this embodiment the arcuate portion includes arcuate spline grooves 40 and the straight portion includes radial straight portions 42 that connect one groove 40 to another. For example, each of the arcuate spline grooves 40 have a starting point 44 and an end point 46, wherein the starting point 44 starts at a smaller radius than the end point 46. Each of the radial straight portions 42 connects the starting point 44 of one groove 40 to the end point 46 of an adjacent groove 40. Here too, the arcuate portions and the straight portions may be joined by rounded corners.

As before, the inner perimeter comprises the drive section of tack 30, that is, the tack is placed on a complementary shaped drive shaft 48 (Fig. 2A) which turns the tack in order to screw it into tissue (not shown). Tack 30 may also have the steps formed on the upper surface thereof.

It will be appreciated by persons skilled in the art that the present invention is not limited by what has been particularly shown and described hereinabove. Rather the scope of the present invention includes both combinations and subcombinations of the features described hereinabove as well as modifications and variations thereof which would occur to a person of skill in the art upon reading the foregoing description and which are not in the prior art.

## Claims

1. A rotary tack (10) comprising:
a helical body (12) constructed of a resorbable material and having a closed-loop base (14) and a helix of spiral coils (16) that extend from said base (14) which terminates in a tip (18) for piercing tissue, wherein each of said coils (16) has a cross-section with an inner perimeter comprising an arcuate portion (20) and a straight portion (22) contiguous to each other,
**characterised in that** said arcuate portion (20) comprises arcuate spline grooves (40) and said straight portion comprises radial straight portions (42) that connect one groove to another, and each of said arcuate spline grooves (40) has a starting point (44) and an end point (46), wherein the starting point (44) starts at a smaller radius than the end point (46), and each of said radial straight portions (42) connects the starting point (44) of one groove to the end point (46) of an adjacent groove.

2. The rotary tack (10) according to claim 1, wherein said inner perimeter comprises two straight portions (22) that subtend an angle corresponding to two sides of a regular polygon and a single arcuate portion (20) that extends from end points of the two straight portions (22).

3. The rotary tack (10) according to claim 2, wherein said regular polygon is a pentagon.

4. The rotary tack (10) according to claim 1, wherein said arcuate portion (20) and said straight portion (22) are joined by a rounded corner.

5. The rotary tack (10) according to claim 2, wherein said arcuate portion (20) and said straight portions (22) are joined by rounded corners (24) and the two straight portions (22) are joined by a rounded corner (26).

6. The rotary tack (10) according to claim 1, wherein an upper surface of said helix proximate said tip (18) comprises one or more steps (28).

## Patentansprüche

1. Dreh-Reißnagel (10), umfassend:
einen schraubenförmigen Körper (12), der aus einem resorbierbaren Material konstruiert ist und eine Basis (14) mit geschlossener Schleife und eine Wendel aus spiralförmigen Windungen (16), die sich von der Basis (14) erstrecken, aufweist, welche Wendel in einer Spitze (18) zum Durchstechen von Gewebe endet, wobei jede der Windungen (16) einen Querschnitt mit einem Innenumfang aufweist, der einen bogenförmigen Teil (20) und einen geraden Teil (22), die aneinander angrenzen, umfasst,
**dadurch gekennzeichnet, dass** der bogenförmige Teil (20) bogenförmige Keilnuten (40) umfasst und der gerade Teil radiale gerade Teile (42), die eine Nut mit einer anderen verbinden, umfasst, und jede der bogenförmigen Keilnuten (40) einen Anfangspunkt (44) und einen Endpunkt (46) aufweist, wobei der Anfangspunkt (44) an einem kleineren Radius beginnt als der Endpunkt (46), und jeder der radialen geraden Teile (42) den Anfangspunkt (44) einer Nut mit dem Endpunkt (46) einer benachbarten Nut verbindet.

2. Dreh-Reißnagel (10) nach Anspruch 1, wobei der Innenumfang zwei gerade Teile (22), die einem Winkel, der zwei Seiten eines regelmäßigen Vielecks entspricht, gegenüberliegen, und einen einzigen bogenförmigen Teil (20), der sich von Endpunkten der zwei geraden Teile (22) erstreckt, umfasst.

3. Dreh-Reißnagel (10) nach Anspruch 2, wobei das regelmäßige Vieleck ein Fünfeck ist.

4. Dreh-Reißnagel (10) nach Anspruch 1, wobei der bogenförmige Teil (20) und der gerade Teil (22) durch eine abgerundete Ecke verbunden sind.

5. Dreh-Reißnagel (10) nach Anspruch 2, wobei der bogenförmige Teil (20) und die geraden Teile (22) durch abgerundete Ecken (24) verbunden sind und die zwei geraden Teile (22) durch eine abgerundete Ecke (26) verbunden sind.

6. Dreh-Reißnagel (10) nach Anspruch 1, wobei eine Oberseite der Wendel proximal zu der Spitze (18) eine oder mehrere Stufen (28) umfasst.

## Revendications

1. Agrafe rotative (10) comprenant :
un corps hélicoïdal (12) constitué d'un matériau résorbable et possédant une base en boucle fermée (14) et une hélice d'enroulements en spirale (16) qui s'étendent à partir de ladite base (14), qui se termine en une pointe (18) pour transpercer un tissu, dans lequel chacun desdits enroulements (16) possède une section transversale dont le périmètre interne comprend une portion arquée (20) et une portion droite (22) réciproquement contiguës ;
**caractérisé en ce que** ladite portion arquée (20) comprend des rainures arquées (40) en forme de cannelures et ladite portion droite comprend des portions droites radiales (42) qui relient une rainure à l'autre, et chacune desdites rainures arquées (40) en forme de cannelures possède un point de départ (44) et un point final (46), le point de départ (44) démarrant à un rayon inférieur à celui du point final (46), et chacune desdites portions droites radiales (42) reliant le point de départ (44) d'une rainure au point final (46) d'une rainure adjacente.

2. Agrafe rotative (10) selon la revendication 1, dans lequel ledit périmètre interne comprend deux portions droites (22) qui sous-tendent un angle correspondant à deux côtés d'un polygone régulier et une portion arquée unique (20) qui s'étend à partir des points terminaux des deux portions droites (22).

3. Agrafe rotative (10) selon la revendication 2, dans lequel ledit polygone régulier est un pentagone.

4. Agrafe rotative (10) selon la revendication 1, dans lequel ladite portion arquée (20) et ladite portion droite (22) sont jointes par un coin arrondi.

5. Agrafe rotative (10) selon la revendication 2, dans lequel ladite portion arquée (20) et lesdites portions droites (22) sont jointes par des coins arrondis (24) et les deux portions droites (22) sont jointes par un coin arrondi (26).

6. Agrafe rotative (10) selon la revendication 1, dans lequel une surface supérieure de ladite hélice à proximité de ladite pointe (18) comprend une ou plusieurs étapes (28).
